# EUROPEAN PATENT SPECIFICATION

(11) **EP 3 278 830 B1**
(45) Date of publication and mention of the grant of the patent: **15.07.2020**
(21) Application number: 16772508.4
(22) Date of filing: 23.03.2016
(51) Int. Cl.: A61M 5/32, A61M 5/31, A61M 5/315

(54) **ASSEMBLY FOR PROTECTING PUNCTURE NEEDLE, SYRINGE ASSEMBLY, AND METHOD FOR MANUFACTURING SAME**
ANORDNUNG ZUM SCHUTZ EINER PUNKTIONSNADEL, SPRITZENANORDNUNG UND VERFAHREN ZUR HERSTELLUNG DAVON
ENSEMBLE DE PROTECTION D'AIGUILLE DE PONCTION, ENSEMBLE DE SERINGUE ET SON PROCÉDÉ DE FABRICATION

(30) Priority: 30.03.2015 JP 2015068169
(43) Date of publication of application: 07.02.2018
(73) Proprietor: Terumo Kabushiki Kaisha, Tokyo 151-0072 (JP)
(72) Inventor: TAKEMOTO, Masafumi, Fujinomiya-shi Shizuoka 418-0004 (JP)
(74) Representative: Casalonga
(86) International application number: PCT/JP2016/059274
(87) International publication number: WO 2016/158627

(56) References cited:
- WO-A1-2015/022787
- WO-A1-2015/022787
- JP-A- 2014 534 033
- US-A- 4 894 055
- US-A1- 2013 123 711
- US-A1- 2014 257 200
- US-A1- 2015 018 773
- US-A1- 2015 018 773
- US-B2- 6 719 732
- US-B2- 6 719 732

## Description

### Technical Field

The present invention relates to a puncture needle protection assembly configured to protect a puncture needle held by a needle holding portion of a barrel forming a syringe, a syringe assembly to which the puncture needle protection assembly is mounted, and a manufacturing method thereof.

### Background Art

A prefilled syringe, which is provided in the state of being prefilled with a liquid drug has been known as a kind of a syringe . In this kind of the prefilled syringe, a protection device, configured to prevent careless puncture by an exposed puncture needle (hereinafter, simply referred to also as a "needle"), is provided in some cases. For example, JP 2013-519415 A discloses a protection device that includes a needle cover body and an outer cylinder member formed using a hollow body of which both ends in a longitudinal direction are open ends and covering the needle cover body from a middle portion in the longitudinal direction thereof to one end portion.

In addition, there is also a technique in which a guide passage is formed in an outer cylinder member, and an inner member formed with a projection to be movably engaged with the guide passage and a spring member biasing the outer cylinder member are housed inside the outer cylinder member as described in the specification of US 2015/0018773 A. According to this configuration, it is possible to cause the outer cylinder member to be biased by the spring member to be relatively moved to a position to surround a needle after puncturing the needle into a patient. Accordingly, the needle is covered by the outer cylinder member, and thus, it is possible to prevent careless puncture.

### Summary of Invention

### Technical Problem

In the configuration described in the specification of US 2015/0018773 A, it is necessary to sequentially attach the spring member, a needle cover body, and the outer cylinder member after assembling the inner member to a barrel portion (barrel). Therefore, it is difficult to assemble the protection device in a place where it is difficult to supply the barrel.

As a result, it is difficult to assemble the protection device alone in the related art. Thus, an assembly facility to assemble the protection device to the barrel is required so that a problem of a surge in capital investment is obvious. Further needle protection devices are disclosed in: WO 2015/022787 A1, US 2013/123711 A1 and US 6 719 732 B2.

A general object of the present invention is to provide a puncture needle protection assembly that enables easy assembly without being assembled with a barrel.

Amain object of the present invention is to provide a puncture needle protection assembly capable of reducing capital investment.

Another object of the present invention is to provide a syringe assembly configured to include the above-described puncture needle protection assembly.

Still another object of the present invention is to provide a method of manufacturing the syringe assembly.

### Solution to Problem

According to one embodiment of the present invention, provided is a puncture needle protection assembly configured to be mounted to a syringe that comprises: a barrel portion configured to be filled with a liquid drug; a needle holding portion that is provided at a distal end of the barrel portion; and a hollow puncture needle that is held by the needle holding portion and includes a distal end projecting distal of the needle holding portion and a proximal end communicating with the inside of the barrel portion, and configured to protect the puncture needle, the puncture needle protection assembly including:
an inner member that is configured to be mountable to the needle holding portion and includes a cam projection and an abutment portion;
an outer cylinder member formed of a hollow body that includes a side wall forming a through hole extending from a distal end to a proximal end, a guide passage formed in the side wall, a stepped portion formed in the side wall, and a central axis, the inner member configured to be accommodated in the through hole, the cam projection of the inner member configured to be movable in the guide passage, and the outer cylinder member configured to be movable relative to the inner member along the central axis;
a needle cover body formed of a cylindrical body that includes a closed distal end and an opened proximal end, the needle cover body configured to cover the puncture needle and seal the distal end of the puncture needle, and including a part accommodated in the through hole and a distal end exposed from the through hole; and
a biasing member that is accommodated in the through hole and biases the outer cylinder member with respect to the inner member,
wherein, when the puncture needle protection assembly is attached to the syringe, the outer cylinder member is movable to an initial position to cover at least a part of the puncture needle, a proximal end position to expose the part of the puncture needle by relatively moving in a proximal end direction with respect to the inner member, and a locking position to cover the distal end of the puncture needle by moving from the proximal end position in a distal end direction with respect to the inner member,
wherein the inner member is configured to rotate relative to the outer cylinder member by movement of the cam projection in the guide passage while the outer cylinder member moves from the initial position to the locking position, and configured to restrict a movement of the outer cylinder member in the proximal direction with respect to the inner member when the outer cylinder member is moved to the locking position, and
wherein the needle cover body includes a pressing portion that is configured to push the inner member toward the needle holding portion by abutting on the abutment portion of the inner member, and a hook portion that is configured to be hooked by the stepped portion of the outer cylinder member.

In this configuration, the biasing member is interposed between the inner member and the outer cylinder member. In this state, the hook portion of the needle cover body is hooked by an inner wall of the outer cylinder member. Such hooking serves to prevent the needle cover body from slipping out of the outer cylinder member. In addition, since the cam projection of the inner member is arranged in the guide passage of the outer cylinder member, the inner member is held in the outer cylinder member. Therefore, even if the biasing member applies a biasing force to the inner member or the outer cylinder member, the needle cover body and the inner member are prevented from being detached from the through hole of the outer cylinder member.

For the above-described reasons, the puncture needle protection assembly can be maintained in such a state . Therefore, the puncture needle protection assembly can be assembled alone without being assembled with the syringe. Thus, for example, it is possible to assemble the puncture needle protection assembly at a predetermined place, and then, to transport the puncture needle protection assembly to another place and attach the puncture needle protection assembly to the syringe, and the like.

Moreover, when attaching the puncture needle protection assembly to the syringe, it is sufficient to perform simple work of puncturing the needle cover body with a needle. That is, equipment for assembling the puncture needle protection assembly with the syringe is unnecessary. Therefore, it is possible to achieve reduction in capital investment.

In the above-described configuration, it is preferable that at least one of the stepped portion and the hook portion be elastically deformable . In this case, at least one of the stepped portion or the hook portion elastically deforms as the needle cover body is pushed into the outer cylinder member. Therefore, it is easy to push the needle cover body into the outer cylinder member.

The needle cover body is configured only of a punctured member that is punctured with the puncture needle, but the punctured member is generally soft and is sometimes hardly pushed in the outer cylindrical member or pulled out from the outer cylindrical member. Thus, the punctured member is formed of an elastic body that includes a punctured portion configured to be punctured with the distal end of the puncture needle and a covering portion extending from the punctured portion in the proximal direction so as to an outer surface of the puncture needle, and to mount a rigid member having higher rigidity than the punctured member on the outside of the punctured member. The pressing portion is provided in the rigid member.

In this case, the rigid member provides a certain degree of rigidity to the needle cover body. Therefore, it is possible to smoothly advance the needle cover body to be pushed into the outer cylinder member or to be pulled out from the outer cylinder member. Moreover, it is easy to push the inner member since the rigid member having high rigidity abuts on the abutment portion of the inner member.

The puncture needle protection assembly together is sometimes subjected to sterilization treatment with the syringe . The sterilization treatment is generally performed by high-pressure steam. Here, an object having high rigidity usually has low water vapor permeability. Thus, it is preferable to configure the rigid member to include a plurality of columnar portions extending along the central axis direction and a slit formed between two adj acent columnar portions among the plurality of columnar portions so as to expose the punctured member.

Since the punctured member is exposed from the slit, water vapor easily contacts the punctured member. Thus, the water vapor easily permeates into the punctured member so that it is possible to perform the sterilization treatment on the punctured member and the puncture needle.

Incidentally, the hook portion may be formed on an outer surface of the columnar portion in this case. In this configuration, the hook portion is likely to be elastically deformed inward. Therefore, it becomes easy to insert and hold the needle cover body inside the outer cylinder member. In other words, it becomes easy to fit the needle cover body to the outer cylinder member.

In addition, the punctured member may include a close contact portion that is configured to be brought into close contact with a distal end of the needle holding portion at a proximal end of the covering portion. In this case, when the puncture needle protection assembly is mounted to the syringe, the punctured member is compressed between the distal end of the needle holding portion and the hook portion. As a result, the close contact portion forms an airtight seal for sealing the covering portion with the distal end of the needle holding portion.

A first engaging portion may be formed on the inner member and a second engaging portion to be engaged with the first engaging portion along the central axis may be formed on the needle cover body. In this case, connection strength between the inner member and the needle cover body becomes favorable, and it is easy to move the inner member and the needle cover body in an integrated manner.

In addition, axes of the needle cover body and the inner member substantially match each other. Thus, there is no risk that the needle cover body is inclined with respect to the inner member when being mounted to the syringe so that the puncture needle is bent due to the puncture in an inclined posture or projects from a side of the needle cover body.

The hook portion formed in the needle cover body may include a tapered portion which projects from an outer surface of the needle cover body and of which projection height gradually increases toward a distal end. In this case, when the hook portion is inserted into the through hole, the hook portion easily deflects inward as such insertion proceeds. That is, it is easy to insert a part where the hook portion is provided into the through hole. Further, the hook portion after being inserted into the through hole returns to the original position by elastic action, and the distal end of the tapered portion is hooked by a proximal end of the stepped portion.

The stepped portion is provided, for example, on an inner surface of the side wall of the outer cylinder member. On the other hand, at least three hook portions may be formed on the outer surface of the needle cover body and may include a support portion that abuts on an inner peripheral wall of the stepped portion.

Accordingly, it is possible to suppress the inclination of the needle cover body with respect to the outer cylinder member. Therefore, there is no risk that the needle cover body is inclined with respect to the inner member when being mounted to the syringe so that the puncture needle is bent due to the puncture in the inclined posture or projects from the side of the needle cover body.

Further, it is preferable to configure the stepped portion to include a distal end seat portion which projects from the side wall in the vicinity of a distal end opening of the outer cylinder member and supports a distal end of the biasing member at an outer side than the part to be engaged with the hook portion. Accordingly, it is possible to avoid mutual interference between the biasing member and the needle cover body.

Further, a flange portion, which projects in a direction orthogonal to the central axis, may be formed in a part of the needle cover body exposed from the through hole. When the needle cover body is pushed into the through hole or pulled out from the through hole, the user may grip this flange portion. Accordingly, it is possible to easily perform the push-in work and the pull-out work.

According to another embodiment of the present invention, provided is a syringe assembly including: the puncture needle protection assembly configured as described above; and a syringe to which the puncture needle protection assembly is mounted.

A preferred example of the syringe is a prefilled syringe. In this case, the prefilled syringe includes a liquid drug filled in the barrel portion, a gasket slidably inserted in the barrel portion, and a pusher to operate the gasket.

According to still another embodiment of the present invention, provided is a method of manufacturing a syringe assembly to obtain the syringe assembly by mounting the puncture needle protection assembly configured as described above to a syringe, the method of manufacturing the syringe assembly including:
assembling the puncture needle protection assembly in advance before mounting the inner member to the needle holding portion;
causing a proximal end of the puncture needle protection assembly to approach the syringe from a distal end side of the syringe; and
pressing the needle cover body in the proximal end direction to push the inner member toward the needle holding portion via an abutment between the pressing portion and the abutment portion.

As described above, it is configured such that the hook portion of the needle cover body accommodated in the through hole of the outer cylinder member is hooked by the inner wall of the outer cylinder member, and the cam projection of the inner member is arranged in the guide passage formed in the outer cylinder member. Thus, the needle cover body is prevented from slipping out of the outer cylinder member, and the inner member is retained in the outer cylinder member. Therefore, even if the biasing member interposed between the inner member and the outer cylinder member applies the biasing force to the inner member or the outer cylinder member, the needle cover body and the inner member are prevented from being detached from the through hole of the outer cylinder member.

Thus, it is possible to maintain the puncture needle protection assembly in a state where the needle cover body is prevented from slipping out of the outer cylinder member and the inner member is retained in the outer cylinder member. That is, it is possible to assemble the puncture needle protecting assembly alone without being assembled with the syringe.

Therefore, the equipment for assembling the puncture needle protection assembly with the syringe is unnecessary. Accordingly, it is possible to achieve the reduction in capital investment.

### Brief Description of Drawings

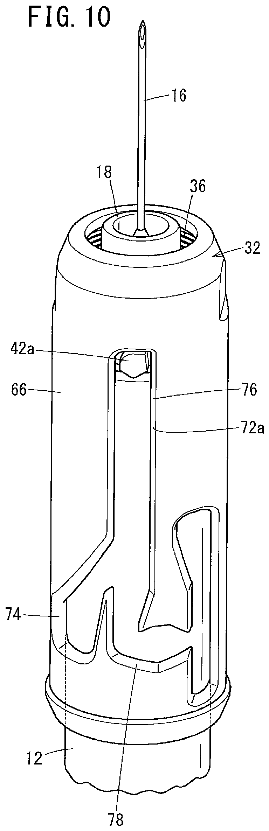

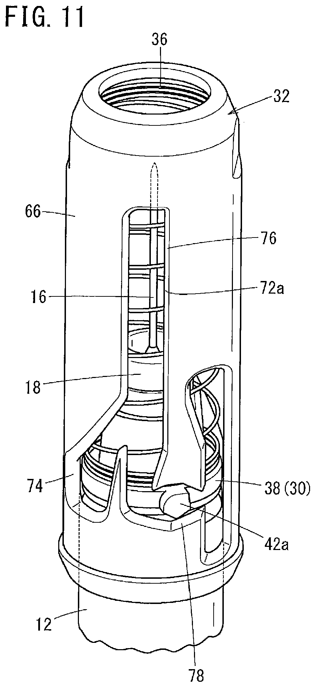

Fig. 1 is an overall schematic cross-sectional view of a syringe assembly including a puncture needle protection assembly according to an embodiment of the invention.
Fig. 2 is a schematic perspective view of a main part when the puncture needle protection assembly according to the embodiment of the present invention is mounted to the syringe.
Fig. 3 is a schematic cross-sectional view in a longitudinal direction of a main part when the puncture needle protection assembly of Fig. 2 is mounted to the syringe.
Fig. 4 is an exploded perspective view illustrating the puncture needle protection assembly of Fig. 2 together with the syringe.
Fig. 5 is an exploded perspective view illustrating a state where a needle cover body is inserted into an outer cylinder in order to assemble the puncture needle protection assembly of Fig. 2.
Fig. 6 is a vertical cross-sectional view of Fig. 5.
Continuously to Fig. 5 and Fig. 6, Fig. 7 is a vertical cross-sectional view illustrating a state where the needle cover body is inserted into the outer cylinder.
Continuously to Fig. 7, Fig. 8 is a vertical cross-sectional view illustrating a state when the puncture needle protection assembly is mounted to the syringe.

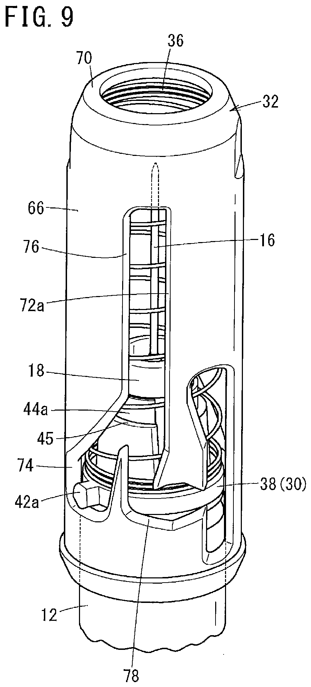

Fig. 9 is a perspective view of a main part illustrating a state where the needle cover body is detached from an inside of the outer cylinder.
Continuously to Fig. 9, Fig. 10 is a perspective view of a main part illustrating a state where the puncture needle is exposed from the outer cylinder.
Continuously to Fig. 10, Fig. 11 is a perspective view of a main part illustrating a state where the puncture needle is accommodated in the outer cylinder.

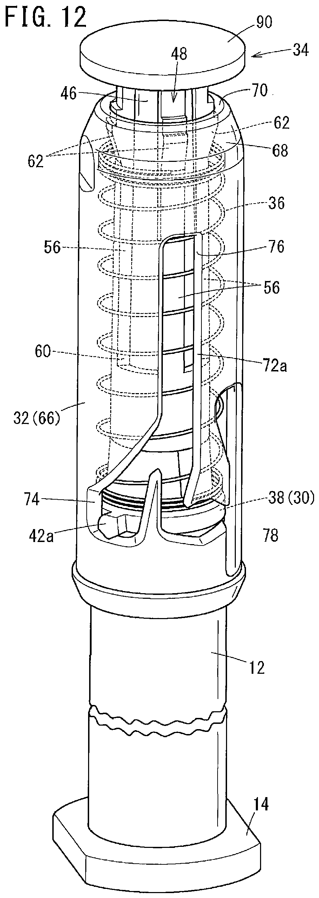

Fig. 12 is a schematic perspective view of a main part when the puncture needle protection assembly according to the embodiment of the present invention is mounted to the syringe.

### Description of Embodiments

Hereinafter, preferred embodiments regarding a puncture needle protection assembly according to the present invention in relation to a syringe assembly and a manufacturing method thereof will be described in detail with reference to the accompanying drawings. Incidentally, a "user" in the following description indicates a person who administers a liquid drug (injection solution) to a patient, but the user is not limited to a doctor, a nurse, and the like, but also includes the patient himself.

In addition, a "proximal end" in the following description indicates an end on a side close to the user who operates the syringe, and a "distal end" indicates an end on a side spaced apart from the user.

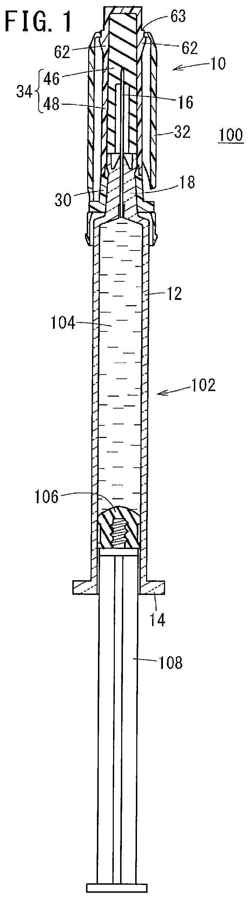

Fig. 1 is an overall schematic sectional view of a syringe assembly 100 according to the present embodiment. The syringe assembly 100 includes a prefilled syringe 102 as a syringe and a puncture needle protection assembly 10 mounted to the prefilled syringe 102.

The prefilled syringe 102 will be briefly described. The prefilled syringe 102 includes a hollow barrel 12 which is a barrel portion, and the barrel 12 is filled with a liquid drug 104. The proximal end side of the barrel 12 is opened, and a gasket 106 is inserted into the barrel 12 from this opened proximal end side. That is, the proximal end side of the barrel 12 is sealed by the gasket 106, and the liquid drug 104 is sealed in the barrel 12.

A pusher 108 is connected to the gasket 106. When the user presses the pusher 108, the gasket 106 slides inside the barrel 12. Incidentally, the pusher 108 punctures the patient with a puncture needle 16, and is connected to the gasket 106 when the liquid drug 104 is administered to the patient.

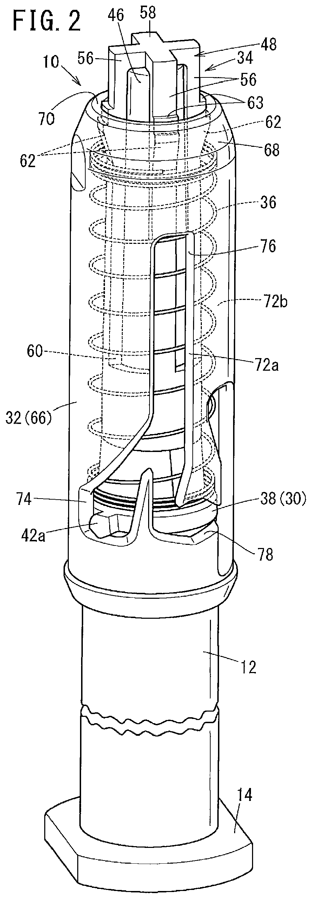

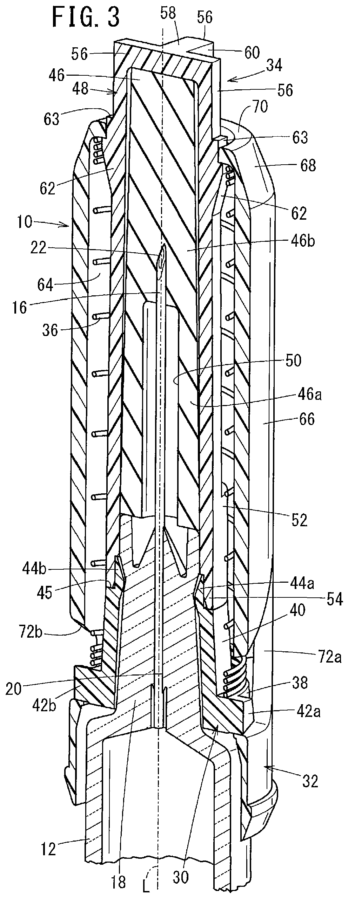

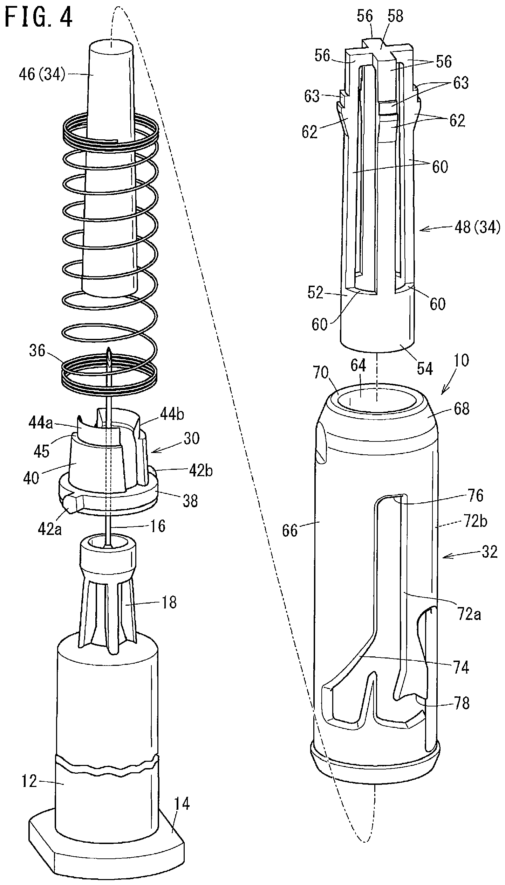

Figs. 2 and 3 are a schematic perspective view of a main part and a schematic cross-sectional view in a longitudinal direction of a main part, respectively, when the puncture needle protection assembly 10 according to the present embodiment is mounted to the barrel 12 of the prefilled syringe 102, and Fig. 4 is an exploded perspective view illustrating the puncture needle protection assembly 10 together with the barrel 12. First, the barrel 12 will be briefly described.

As understood particularly from Fig. 3, the barrel 12 is a hollow body having a substantially cylindrical shape and having one opened end, and a hook portion 14 (see Figs. 2 and 4) on which a finer is hooked when the user presses the pusher 108 (not illustrated) is formed on a side wall of the one opened end so as to project outward in a diameter direction. In addition, a needle holding portion 18, which extends along a longitudinal direction of the barrel 12 and holds the puncture needle (needle) 16, is formed on an outer wall of the other closed end.

A holding hole 20, which has an inner diameter corresponding to an outer diameter of the needle 16 and extends from a distal end portion to the hollow inside of the barrel 12, is formed in an axial center portion of the needle holding portion 18. The needle 16 is fixed and held in the holding hole 20 by an appropriate fixing method such as insert molding, heat welding using a high frequency or a laser, and adhesion using an adhesive. The needle 16 is a hollow body in which a lead-out passage 22 is formed, and the liquid drug 104 contained in the hollow inside of the barrel 12 is discharged from a distal end of the lead-out passage 22 and is administered to the patient.

The puncture needle protection assembly 10 is configured to prevent unnecessary puncture other than the time when the liquid drug 104 is administered to the patient by covering the needle 16 before and after puncturing the patient with the needle 16. Next, the puncture needle protection assembly 10 will be described in detail.

As illustrated in Figs. 2 to 4, the puncture needle protection assembly 10 includes a hollow inner cylinder 30 (inner member), an outer cylinder 32 (outer cylinder member) accommodating the inner cylinder 30 therein, a needle cover body 34 covering the puncture needle 16, and a spring member 36 as a biasing member that biases the inner cylinder 30 toward the barrel 12.

As illustrated in Fig. 4, the inner cylinder 30 is made of resin and includes a proximal end spring seat 38 having a substantially disc shape and a seat portion 40 having a substantially truncated-cone shape projecting from the proximal end spring seat 38. Two cam projections 42a and 42b are formed on a side wall of the proximal end spring seat 38 so as to project outward in the diameter direction. Meanwhile, a distal end portion of the seat portion 40 is formed to be thin such that two arc-shaped thin portions 44a and 44b (first engaging portions) and an abutment portion 45 as an arc-shaped stepped portion are formed.

The needle cover body 34 is provided continuously to the inner cylinder 30. In the present embodiment, the needle cover body 34 is formed of a shield 46 (a punctured member) that is punctured with the needle 16 and a cover 48 (a rigid member) that is mounted so as to cover the shield 46 from the outside.

The shield 46 is formed using a cylindrical body having a substantially truncated-cone shape of which diameter decreases in a tapered shape toward a side to be spaced apart from a side close to the needle 16. The shield 46 is formed using rubber and is relatively soft since a cavity 50 is formed inside the shield 46. Therefore, it is easy to puncture the shield 46 with the needle 16.

A terminal end on the distal end side of the cavity 50 is set midway in the axial direction of the shield 46. That is, the shield 46 has an opened proximal end and a closed distal end, and thus, has a shape in which a hollow portion is formed on the proximal end side and a solid portion is formed on the distal end side. The hollow portion serves a role as a covering portion 46a that covers an outer surface of the needle 16. In addition, the solid portion is a punctured portion 46b that is punctured with the distal end of the needle 16.

In the shield 46, a proximal end face of the covering portion 46a becomes a close contact portion that can be brought into close contact with a distal end face of the needle holding portion 18. As the proximal end face of the covering portion 46a and the distal end face of the needle holding portion 18 are brought into close contact with each other in this manner, the covering portion 46a (the cavity 50) is sealed and an airtight seal is formed.

The shield 46 is preferably made of a material to which water vapor is permeable. Preferable examples thereof may include silicone rubber, isoprene rubber, butadiene rubber, and the like.

The cover 48 is made of a material having higher rigidity than the shield 46, for example, resin. Therefore, the cover 48 is mounted on the outside of the shield 46 so that the needle cover body 34 is configured to exhibit the sufficient rigidity. Thus, the user can easily push the shield 46 into the outer cylinder 32 via the cover 48 or to detach the shield 46 together with the cover 48 from the needle 16.

A proximal end of the cover 48 is formed of a cylindrical portion 52. An annular thin portion 54 (second engaging portion) is formed in the vicinity of an opening of the cylindrical portion 52 so as to cut out a part of an inner wall of the cylindrical portion 52 outward in the diameter direction. The arc-shaped thin portions 44a and 44b of the inner cylinder 30 are inserted into the annular thin portion 54. Incidentally, the cover 48 is longer than the shield 46, and thus, the annular thin portion 54 is exposed without being covered by the shield 46.

The proximal end face of the annular thin portion 54 abuts on the abutment portion 45 of the inner cylinder 30 to press the inner cylinder 30. That is, the annular thin portion 54 also functions as a pressing portion.

Four columnar portions 56, spaced apart from each other approximately by 90°, project from the cylindrical portion 52 so as to extend along a center axis of the outer cylinder 32, and each distal end portion thereof is connected via a connecting portion 58 having a cross shape . A portion between the adjacent columnar portions 56 and 56 is opened as a slit 60, and thus, a side wall of the shield 46 can be visually recognized through the slit 60. That is, a part of the shield 46 is exposed by the slit 60.

Each of the four columnar portions 56 is formed with a hook portion 62 including a tapered portion of which projection height gradually increases as approaching the connecting portion 58, in other words, the distal end from the cylindrical portion 52. In the present embodiment, since the four columnar portions 56 are provided and the single hook portion 62 is provided in each of the columnar portions 56, the four hook portions 62 are formed in total.

The columnar portion 56 has a narrow width, and thus, the columnar portion 56 has sufficient flexibility and elasticity. When the columnar portion 56 is deflected or return to the original position by elastic action, the hook portion 62 is displaced accompanying the columnar portion 56. That is, the hook portion 62 is elastically deformable.

Further, a support portion 63 of which side wall face is parallel to a center axis L of the outer cylinder 32 is formed to project on a side closer to the distal end than the hook portion 62. That is, the four support portions 63 are also provided in total.

A through hole 64 extending along the center axis L is formed in the outer cylinder 32. That is, the outer cylinder 32 is a hollow body of which both ends are opened and includes a side wall 66, a tapered side wall 68 of which diameter decreases in a tapered shape toward the distal end side from the side wall 66, and a stopper wall 70 (stepped portion) slightly projecting inward in the diameter direction from an edge portion of the tapered side wall 68.

As illustrated in Fig. 4, an outer diameter of the side wall 66 is set to be slightly larger than an outer diameter of the barrel 12. Therefore, the barrel 12 is covered by one end of the side wall 66.

As illustrated in Figs. 2 and 4, a pair of guide passages 72a and 72b are formed to be long along the longitudinal direction of the side wall 66 on the distal end side from a substantially middle portion of the side wall 66 in the longitudinal direction. The guide passages 72a and 72b penetrate through the outer cylinder 32 from an inner wall to an outer wall thereof. In addition, the guide passages 72a and 72b are spaced apart from each other by 180° and formed to be rotationally symmetric. Further, the cam projections 42a and 42b of the inner cylinder 30 are movably engaged with the guide passages 72a and 72b, respectively.

The guide passages 72a and 72b are bifurcated at the proximal end side and are merged at the distal end side to form a series of elongated holes extending linearly. Specifically, the guide passages 72a and 72b are formed of a first passage 74, a second passage 76, and a third passage 78 intersecting each other at an intersection. The first passage 74 among them extends linearly from the proximal end toward the distal end, and then, is inclined in a circumferential direction and a distal end direction toward the intersection. The proximal end side of the first passage 74 is a pre-puncture position (initial position) where the cam projections 42a and 42b are positioned before the puncture of the needle 16.

The second passage 76 is a through passage extending linearly in the distal end direction from the intersection. A distal end of the second passage 76 becomes a puncture position (proximal end position) to which the cam projections 42a and 42b move at the time of the puncture of the needle 16. Further, the third passage 78 is a through passage extending linearly in a proximal end direction from the intersection. The proximal end side of the third passage 78 becomes a post-puncture position (locking position) to which the projection moves after the puncture of the needle 16. The first passage 74 and the third passage 78 intersect each other at a predetermined angle (for example, 45° or smaller).

The distal end of the barrel 12, the inner cylinder 30, the needle cover body 34, and the spring member 36 are accommodated inside the through hole 64, that is, the outer cylinder 32. A proximal end of the spring member 36 among them is seated on the distal end face of the proximal end spring seat 38 of the inner cylinder 30, and a distal end thereof is seated on a distal end seat portion which is a proximal end of the stopper wall 70 of the outer cylinder 32. The spring member 36 biases the inner cylinder 30 to be oriented toward the barrel 12, and the hook portion 62 of the needle cover body 34 (cover 48) is hooked by the stopper wall 70 of the outer cylinder 32, and accordingly, the inner cylinder 30 and the needle cover body 34 are prevented from slipping out of the outer cylinder 32.

The stopper wall 70 is set to be sufficiently thin, and thus, has sufficient flexibility. That is, the stopper wall 70 is also elastically deformable. In addition, an inner wall face of the stopper wall 70 abuts on a side wall face of the support portion 63.

The seat portion 40 of the inner cylinder 30 and the most part of the needle cover body 34 are inserted in the spring member 36. An inner diameter of the spring member 36 is set to be larger than an outer diameter of a part of the cover 48 where the hook portion 62 is provided. Therefore, the spring member 36 is not brought into contact with the cover 48 or the needle cover body 34, and the distal end of the spring member 36 is at a position surrounding the hook portion 62 from the outside and is seated on the distal end seat portion of the stopper wall 70.

Further, a protective cylinder (not illustrated) may be mounted to the outer cylinder 32. Inthiscase, engaging portions are provided on the protective cylinder and the outer cylinder 32, respectively, and the protective cylinder may be held on the outer cylinder 32 by engaging the engaging portions with each other.

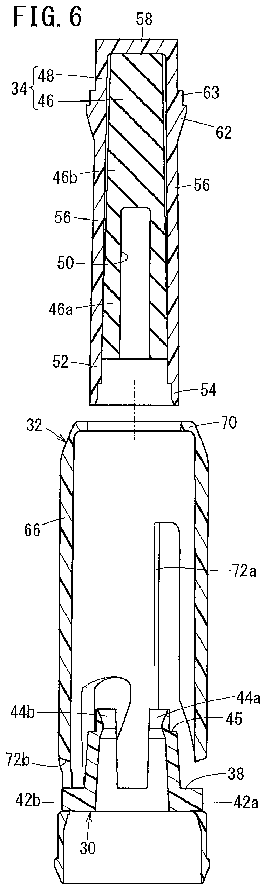

Basically, the puncture needle protection assembly 10 configured as described above can be assembled in the following manner. Incidentally, Figs. 6 to 8 do not illustrate the spring member 36 in order to facilitate the visual recognition of the inside of the outer cylinder 32.

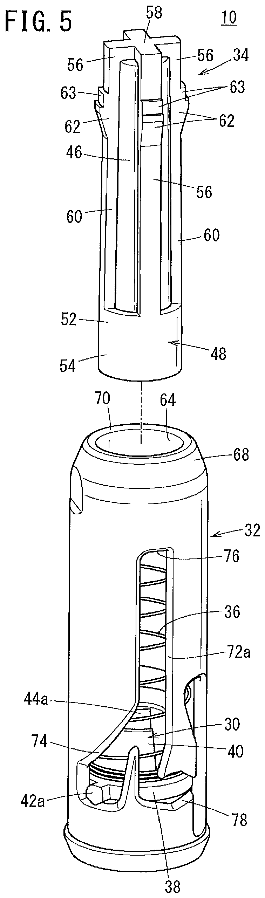

First, the spring member 36 is inserted into the outer cylinder 32 such that the proximal end is seated on the distal end face of the proximal end spring seat 38 and the distal end is seated on the proximal end of the stopper wall 70 of the outer cylinder 32. Thereafter, the inner cylinder 30 is inserted inside the outer cylinder 32 as illustrated in Figs. 5 and 6, and the cam projections 42a and 42b of the inner cylinder 30 are positioned at the proximal end of the first passages 74 of the guide passages 72a and 72b.

On the other hand, the needle cover body 34 is obtained by mounting the cover 48 to the outside of the shield 46. At this time, the shield 46 is pushed into the cover 48 until the distal end face thereof is blocked by the connecting portion 58. The cover 48 is longer than the shield 46 as described above, and thus, the inside of the cylindrical portion 52 of the cover 48 is hollow.

Next, the needle cover body 34 is inserted into the outer cylinder 32 such that the needle cover body 34 passes through a distal end opening of the outer cylinder 32 from the cylindrical portion 52 of the cover 48. Since the spring member 36 is set to have a larger diameter than the cover 48, the spring member 36 does not interfere with the needle cover body 34 at this time. In addition, since the cover 48 has a certain degree of rigidity, it is possible to easily insert the soft shield 46 into the outer cylinder 32, for example, by pressing the connecting portion 58.

During the insertion of the needle cover body 34, an inclined face of the hook portion 62 abuts on the inner wall face of the stopper wall 70. Here, the columnar portion 56 provided with the hook portion 62 and the stopper wall 70 exhibit the sufficient flexibility. Further, the hook portion 62 has the tapered shape such that the projecting height increases toward the distal end. Thus, the hook portion 62 is pressed inward in the diameter direction by the stopper wall 70 when the needle cover body 34 in the state where the inclined face of the hook portion 62 abuts on an inner peripheral face of the stopper wall 70 is further pushed by pressing the connecting portion 58. As a result, the columnar portion 56 deflects inward in the diameter direction, and the hook portion 62 is displaced inward in the diameter direction accompanying the bending columnar portion 56. Incidentally, the shield 46 is soft, and thus, is deformed in accordance with the deflection of the columnar portion 56.

For the above reasons, the insertion of the needle cover body 34 into the outer cylinder 32 is not hindered although the hook portion 62 is provided in the cover 48. That is, the needle cover body 34 is smoothly inserted into the outer cylinder 32, and eventually, the arc-shaped thin portions 44a and 44b enter the inside of the annular thin portion 54 of the cover 48. Accordingly, the annular thin portion 54 is engaged with the arc-shaped thin portions 44a and 44b, and the needle cover body 34 is provided continuously to the inner cylinder 30.

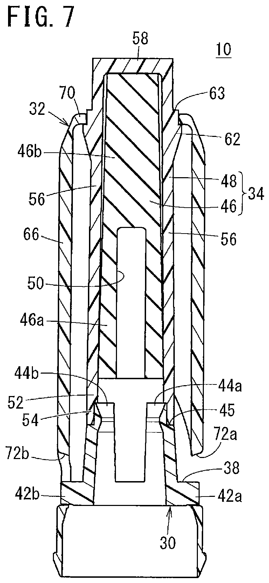

At the same time, the inner cylinder 30 is pressed and pushed toward the needle holding portion 18 as the annular thin portion 54 (pressing portion) abuts on the abutment portion 45 of the inner cylinder 30 as illustrated in Fig. 7. Meanwhile, the proximal end face of the covering portion 46a of the shield 46 is brought into close contact with the distal end face of the needle holding portion 18. That is, the airtight seal is formed between the proximal end face of the covering portion 46a and the distal end face of the needle holding portion 18, thereby sealing the covering portion 46a (cavity 50).

In addition, the hook portion 62 is released from a pressing force when the hook portion 62 passes through the stopper wall 70 and is inserted into the outer cylinder 32. Therefore, the columnar portion 56 returns to the original shape by elastic action, and accordingly, the hook portion 62 is displaced outward in the diameter direction to return to the original position. At this time, the flat distal end face of the hook portion 62 is hooked by a ceiling surface (inner wall) of the stopper wall 70 as illustrated in Figs. 2 and 3. The needle cover body 34 is prevented from slipping out of the outer cylinder 32 by such hooking. Since the hook portion 62 is provided in the middle of the columnar portion 56, an upper part than the hook portion 62 is exposed from the outer cylinder 32, and a sidewall face of the support portion 63 abuts on the inner wall face of the stopper wall 70.

Further, the protective cylinder is mounted to the outer cylinder 32 if necessary. As described above, the puncture needle protection assembly 10 is obtained.

In this manner, it is possible to assemble the puncture needle protection assembly 10 alone according to the present embodiment. That is, it is unnecessary to prepare the barrel 12 when assembling the puncture needle protection assembly 10. Thus, itispossible to distribute the puncture needle protection assembly 10 separately from the barrel 12 (or the syringe), for example, after assembling the puncture needle protection assembly 10. Incidentally, the needle cover body 34 is prevented from slipping out of the outer cylinder 32 as the hook portion 62 is hooked by the stopper wall 70, and thus, the loss of the needle cover body 34 is avoided during the distribution.

It is a matter of course that equipment for assembling the puncture needle protection assembly 10 with the barrel 12 is unnecessary. Therefore, it is also possible to achieve reduction in capital investment.

Next, a method of manufacturing the syringe assembly 100 (see Fig. 1) will be described.

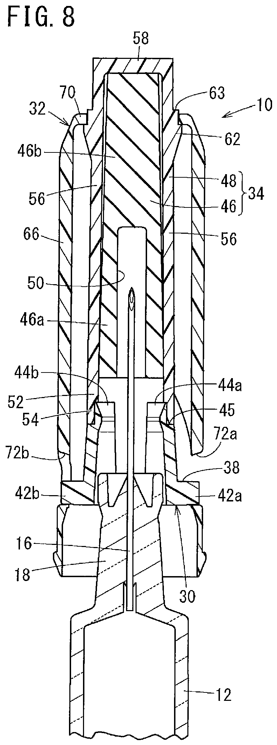

When the syringe is the prefilled syringe 102, the puncture needle protection assembly 10 is attached to the distal end of the barrel 12 as illustrated in Fig. 8. At this time, the needle holding portion 18 is inserted into the inner cylinder 30, and further, enters the cylindrical portion 52 of the cover 48 such that the distal end face abuts on the proximal end face of the shield 46. It is a matter of course that the distal end of the needle 16 passes through the cavity 50 of the shield 46 and with which the punctured portion 46b of the shield 46 is punctured.

Since axial centers of the needle cover body 34 and the inner cylinder 30 substantially match each other and the four hook portions 62 are provided, the inclination of the needle cover body 34 with respect to the inner cylinder 30 is avoided when the puncture needle protection assembly 10 is mounted to the needle 16 and the barrel 12. Therefore, it is possible to prevent the puncture of the puncture needle 16 into the shield 46 in an inclined posture or the projection of the puncture needle 16 from the needle cover body 34.

Thereafter, the barrel 12 is filled with the liquid drug 104, and further, the barrel 12 is sealed by the gasket 106. Accordingly, the syringe assembly 100 in which the puncture needle protection assembly 10 is mounted to the prefilled syringe 102 in which liquid drug 104 is sealed in the barrel 12 is obtained.

In this state, the syringe assembly 100 is accommodated in an autoclave and is subjected to sterilization treatment. Water vapor is introduced into the autoclave during the sterilization treatment. The water vapor performs sterilization with respect to the prefilled syringe 102, passes through the distal end opening of the outer cylinder 32, enters the inside of the outer cylinder 32, and contacts the needle cover body 34. Here, the slit 60 is formed between the columnar portions 56 in the cover 48 forming the needle cover body 34. Thus, the water vapor easily contacts the shield 46 exposed from the slit 60.

The shield 46 is made of rubber (for example, silicone rubber, isoprene rubber, or the like) having excellent water vapor permeability. Thus, the water vapor contacting the shield 46 permeate into the shield 46 and easily reaches the inside of the covering portion 46a and the punctured portion 46b. The water vapor that has reached the punctured portion 46b further enters the lead-out passage 22 through an opening of the lead-out passage 22. Since the lead-out passage 22 communicates with the inside of the barrel 12, the water vapor passes through the lead-out passage 22 and enters the inside of the barrel 12. As described above, the inside of the needle 16 is subjected to the sterilization treatment.

In addition, the water vapor that has entered the cavity 50 of the shield 46 contacts an outer peripheral wall of the needle 16. As a result, the outside of the needle 16 is subjected to the sterilization treatment.

As described above, the slit 60 is formed in the cover 48 covering the shield 46 so as to expose the shield 46 from the slit 60 in the puncture needle protection assembly 10 according to the present embodiment, and thus, it is possible to cause the water vapor to permeate into the shield 46 and to perform the sterilization treatment on the inside and outside of the needle 16.

Conversely to the above description, it may be configured such that the barrel 12 is subjected to the sterilization treatment, thereafter, the barrel 12 is filled with the liquid drug 104, and further, the barrel 12 is sealed by the gasket 106. Even in this case, it is possible to perform the sterilization treatment on the inside and outside of the needle 16 for the same reason as described above.

When the liquid drug 104 is administered to the patient, the user (which may be the patient himself) first connects the pusher 108 to the gasket 106 to obtain the state illustrated in Fig. 1. Incidentally, the syringe assembly 100 may be provided to the user in a state where the pusher 108 is connected to the gasket 106 in advance as illustrated in Fig. 1.

Further, the connecting portion 58 of the cover 48 or the like is gripped, and the needle cover body 34 is pulled up. At this time, the hook portion 62 is pressed by the stopper wall 70 and slightly deflects downward. Accordingly, the hook portion 62 is released from the stopper wall 70, and thus, it is possible to cause the needle cover body 34 to be easily detached from the outer cylinder 32 to obtain the state illustrated in Fig. 9. It is a matter of course that the needle 16 is relatively pulled out from the punctured portion 46b of the shield 46 at the time of such detachment.

At this point in time, the inner cylinder 30 is pressed against the barrel 12 side by biasing of the spring member 36, and thus, the inner cylinder 30 and the outer cylinder 32 are not displaced. Further, the state where the needle 16 is surrounded by the outer cylinder 32 to the distal end is maintained since the outer cylinder 32 is sufficiently longer than the needle 16. Thus, the user or the like is prevented from being carelessly punctured with the needle 16. It is a matter of course that hygiene of the needle 16 is maintained since the contact of the needle 16 with any object is avoided. Incidentally, the cam projections 42a and 42b are kept in the state of being arranged at the pre-puncture position (initial position) since the inner cylinder 30 and the outer cylinder 32 are not displaced.

Next, the user simply positions the distal end portion of the outer cylinder 32 to be in contact with a puncture site (an arm or the like) of the patient and further causes the barrel 12 to gradually move forward relative to the outer cylinder 32. As the barrel 12 moves forward relative to the barrel 12, the needle 16 held by the needle holding portion 18 is exposed from the distal end opening of the outer cylinder 32.

At this time, the inner cylinder 30 through which the needle holding portion 18 is inserted also relatively moves forward together with the barrel 12. At this time, the cam projections 42a and 42b of the inner cylinder 30 move to the distal end side along the first passage 74 from the proximal end (the pre-puncture position) of the first passage 74. Thus, the inner cylinder 30 rotates in the circumferential direction along a side wall of the needle holding portion 18 according to the inclination of the first passage 74. At the same time, the spring member 36 is pressed to contract by the inner cylinder 30 relatively moving forward.

Thereafter, the cam projections 42a and 42b advance from the intersection to the second passage 76 accompanying the relative forward movement of the barrel 12, and move to the puncture position at the distal end of the second passage 76 (that is, the proximal end position where the outer cylinder 32 relatively move to the proximal end side) as illustrated in Fig. 10. At this point in time, the most part of the needle 16 is exposed from the distal end of the outer cylinder 32 such that a body of the patient is punctured therewith. Here, it is preferable to set the needle holding portion 18 to a relatively short length. A reason thereof is that the user can recognize that the advancing amount of the barrel 12 substantially matches the exposing amount of the needle 16 in such a case, and thus, it is possible to perform the puncture by appropriately operating the needle 16.

The user presses the pusher 108 in the state where the patient is punctured with the needle 16. Accordingly, the liquid drug 104 with which the barrel 12 is filled is discharged from the needle 16 via the lead-out passage 22. As a result, the liquid drug 104 is administered to the patient.

After the administration of the liquid drug 104, the outer cylinder 32 is pulled out from the patient together with the syringe. At this time, the spring member 36 expands and biases the inner cylinder 30 toward the barrel 12. Accordingly, the barrel 12 moves backward relative to the outer cylinder 32. In other words, the outer cylinder 32 moves forward relative to the barrel 12. At this time, the cam projections 42a and 42b linearly move from the distal end (the puncture position) of the second passage 76 in the proximal end direction along the second passage 76, and further, enters the third passage 78 via the intersection as illustrated in Fig. 11. The relative forward movement of the outer cylinder 32 is completed as the spring member 36 elastically returns to the original shape and the cam projections 42a and 42b move to the post-puncture position (locking position) which is the proximal end of the third passage 78. It is because the cam projections 42a and 42b are brought into contact with a proximal end edge portion of the third passage 78 at this stage so that the outer cylinder 32 is blocked and hardly performs the relative forward movement any more.

A proximal end edge portion (the pre-puncture position) of the first passage 74 and the proximal end edge portion (the post-puncture position) of the third passage 78 are provided at substantially the same height position with the proximal end of the outer cylinder 32 as a start point. Thus, when the cam projections 42a and 42b are brought into contact with the proximal end edge portion of the third passage 78, the needle 16 is re-surrounded by the outer cylinder 32 up to the distal end. That is, the needle 16 is prevented from being exposed from the outer cylinder 32 even after the puncture. Thus, it is possible to avoid the careless puncture even after the administration of the liquid drug 104 to the patient.

The present invention is defined by the appended claims.

For example, a flange portion 90 as illustrated in Fig. 12 may be provided in a part (distal end portion) of the cover 48 that is exposed from the outer cylinder 32. In this case, the flange portion 90 projects in a direction orthogonal to the longitudinal direction of the needle cover body 34.

The user may grip the flange portion 90 when pushing the needle cover body 34 at the time of inserting the needle cover body 34 into the outer cylinder 32 or pulling the needle cover body 34 out of the outer cylinder 32 so as to puncture the patient with the needle 16. Accordingly, the pushing-in and pulling-out progress more smoothly.

In addition, the needle cover body may be configured only of the shield although the needle cover body 34 is configured by mounting the cover 48 to the shield 46 in this embodiment. In this case, the hook portion 62 may be provided on a side wall of the shield.

In addition, the syringe is not limited to the prefilled syringe 102, and may be filled with the liquid drug 104 after being provided as a product.

## Claims

1. A puncture needle protection assembly (10) configured to be mounted to a syringe (102) that comprises: a barrel portion (12) configured to be filled with a liquid drug (104); a needle holding portion (18) that is provided at a distal end of the barrel portion (12); and a hollow puncture needle (16) that is held by the needle holding portion (18) and includes a distal end projecting distal of the needle holding portion (18) and a proximal end communicating with the inside of the barrel portion (12), and configured to protect the puncture needle (16), the puncture needle protection assembly (10) comprising:
an inner member (30) that is configured to be mountable to the needle holding portion (18), the inner member including a cam projection (42a, 42b) and an abutment portion (45);
an outer cylinder member (32) formed of a hollow body that includes a side wall (66) forming a through hole (64) extending from a distal end to a proximal end, a guide passage (72a, 72b) formed in the side wall (66), a stepped portion (70) formed in the side wall (66), and a central axis, the inner member (30) configured to be accommodated in the through hole (64), the cam projection (42a, 42b) of the inner member (30) configured to be movable in the guide passage (72a, 72b), and the outer cylinder member configured (32) to be movable relative to the inner member (30) along the central axis;
a needle cover body (34) formed of a cylindrical body that includes a closed distal end and an opened proximal end, the needle cover body configured to cover the puncture needle (16) and seal the distal end of the puncture needle (16), and including a part accommodated in the through hole (64) and a distal end exposed from the through hole (64); and
a biasing member (36) that is accommodated in the through hole (64) and biases the outer cylinder member (32) with respect to the inner member (30),
wherein, when the puncture needle protection assembly (10) is attached to the syringe (102), the outer cylinder member (32) is movable to an initial position to cover at least a part of the puncture needle (16), a proximal end position to expose the part of the puncture needle (16) by relatively moving in a proximal end direction with respect to the inner member (30), and a locking position to cover the distal end of the puncture needle (16) by moving from the proximal end position in a distal end direction with respect to the inner member (30),
wherein the inner member (30) is configured to rotate relative to the outer cylinder member (32) by movement of the cam projection (42a, 42b) in the guide passage (72a, 72b) while the outer cylinder member (32) moves from the initial position to the locking position, and configured to restrict a movement of the outer cylinder member (32) in the proximal direction with respect to the inner member (30) when the outer cylinder member (32) is moved to the locking position, and
wherein the needle cover body (34) includes a pressing portion (54) that is configured to push the inner member (30) toward the needle holding portion (18) by abutting on the abutment portion (45) of the inner member (30),
**characterized in that**
a hook portion (62) that is configured to be hooked by the stepped portion (70) of the outer cylinder member (32), and
the needle cover body (34) comprises a punctured member (46) formed of an elastic body that includes a punctured portion (46b) configured to be punctured with the distal end of the puncture needle (16) and a covering portion (46a) extending from the punctured portion (46b) in the proximal direction so as to cover an outer surface of the puncture needle (16); and a rigid member (48) that is configured to be mounted to an outside of the punctured member (46) and includes higher rigidity than the punctured member (46), and the pressing portion (54) is provided in the rigid member (48).

2. The puncture needle protection assembly (10) according to claim 1, wherein at least one of the stepped portion (70) and the hook portion (62) is elastically deformable.

3. The puncture needle protection assembly (10) according to claim 1 or 2, wherein the rigid member (48) includes a plurality of columnar portions (56) extending along the central axis direction and a slit (60) formed between two adjacent columnar portions among the plurality of columnar portions (56) so as to expose the punctured member (46).

4. The puncture needle protection assembly (10) according to claim 3, wherein the hook portion (62) is formed on an outer surface of the columnar portion (56).

5. The puncture needle protection assembly (10) according to any one of claims 1 to 4, wherein the punctured member (46) includes a close contact portion at a proximal end of the covering portion (46a), the close contact portion configured to be brought into close contact with a distal end of the needle holding portion (18), and
wherein, when the puncture needle protection assembly (10) is mounted to the syringe (102), the punctured member (46) is compressed between the distal end of the needle holding portion (18) and the hook portion (62) such that an airtight seal for sealing the covering portion (46a) is formed between the close contact portion and the distal end of the needle holding portion (18).

6. The puncture needle protection assembly (10) according to any one of claims 1 to 5, wherein
a first engaging portion (44a, 44b) is formed on the inner member (30), and
a second engaging portion (54) is formed on the needle cover body (34), the second engaging portion engaged with the first engaging portion (44a, 44b) along the central axis.

7. The puncture needle protection assembly (10) according to any one of claims 1 to 6, wherein
the hook portion (62) includes a tapered portion that projects from an outer surface of the needle cover body (34) and of which projection height gradually increases toward a distal end, and
a distal end of the tapered portion is configured to be hooked by a proximal end of the stepped portion (70).

8. The puncture needle protection assembly (10) according to any one of claims 1 to 7, wherein
the stepped portion (70) is provided on an inner surface of the side wall (66) of the outer cylinder member (32), and
the hook portion (62) forms as at least three hook portions on the outer surface of the needle cover body (34) and includes a support portion (63) that abuts on an inner peripheral wall of the stepped portion (70).

9. The puncture needle protection assembly (10) according to any one of claims 1 to 8, wherein
the stepped portion (70) includes a distal end seat portion that projects from the side wall (66) in the vicinity of a distal end opening of the outer cylinder member (32), the distal end seat portion supporting a distal end of the biasing member (36) at an outer side than a part to be engaged with the hook portion (62).

10. The puncture needle protection assembly (10) according to any one of claims 1 to 9, wherein
a flange portion (90) is formed at a part of the needle cover body (34) exposed from the through hole (64), the flange portion projecting in a direction orthogonal to the central axis.

11. A syringe assembly (100) comprising:
the puncture needle protection assembly (10) according to any one of claims 1 to 10; and
a syringe (102) to which the puncture needle protection assembly (10) is mounted.

12. The syringe assembly (100) according to claim 11, wherein
the syringe (102) is a prefilled syringe (102) that includes:
a liquid drug (104) filled in the barrel portion (12);
a gasket (106) slidably inserted in the barrel portion (12); and
a pusher (108) to operate the gasket (106).

13. A method of manufacturing a syringe assembly (100) to obtain the syringe assembly (100) of claim 11 by mounting the puncture needle protection assembly (10) according to any one of claims 1 to 10 to a syringe (102), the method of manufacturing the syringe assembly (100) comprising:
assembling the puncture needle protection assembly (10) in advance before mounting the inner member (30) to the needle holding portion (18);
causing a proximal end of the puncture needle protection assembly (10) to approach the syringe (102) from a distal end side of the syringe (102);
**characterized by**
pressing the needle cover body (34) in the proximal end direction to push the inner member (30) toward the needle holding portion (18) via an abutment between the pressing portion (54) of the rigid member (48) and the abutment portion (45).

## Patentansprüche

1. Punktionsnadel-Schutzanordnung (10), die konfiguriert ist, um an einer Spritze (102) angebracht zu werden, welche umfasst: einen Zylinderabschnitt (12), der konfiguriert ist, um mit einem flüssigen Arzneimittel (104) gefüllt zu werden; einen Nadelhalteabschnitt (18), der an einem distalen Ende des Zylinderabschnitts (12) vorgesehen ist; und eine hohle Punktionsnadel (16), die von dem Nadelhalteabschnitt (18) gehalten wird und die ein distales Ende, das distal von dem Nadelhalteabschnitt (18) hervorsteht, und ein proximales Ende, das mit der Innenseite des Zylinderabschnitts (12) in Verbindung steht, umfasst und die so konfiguriert ist, dass sie die Punktionsnadel (16) schützt, wobei die Punktionsnadel-Schutzanordnung (10) umfasst:
ein inneres Element (30), das so konfiguriert ist, dass es an dem Nadelhalteabschnitt (18) anbringbar ist, wobei das innere Element einen Nockenvorsprung (42a, 42b) und einen Anschlagabschnitt (45) aufweist;
ein äußeres Zylinderelement (32), das aus einem Hohlkörper gebildet ist, der eine Seitenwand (66), die ein Durchgangsloch (64) bildet, welches sich von einem distalen Ende zu einem proximalen Ende erstreckt, einen in der Seitenwand (66) gebildeten Führungskanal (72a, 72b), einen abgestuften Abschnitt (70), welcher in der Seitenwand (66) ausgebildet ist, und eine Mittelachse aufweist, wobei das innere Element (30) konfiguriert ist, um in dem Durchgangsloch (64) untergebracht zu werden, wobei der Nockenvorsprung (42a, 42b) des inneren Elements (30) so konfiguriert ist, dass er in dem Führungskanal (72a, 72b) beweglich ist, und wobei das äußere Zylinderelement (32) so konfiguriert ist, dass es relativ zu dem inneren Element (30) entlang der Mittelachse beweglich ist;
einen Nadelschutzkappenkörper (34), der aus einem zylindrischen Körper gebildet ist, welcher ein geschlossenes distales Ende und ein offenes proximales Ende aufweist, wobei der Nadelschutzkappenkörper so konfiguriert ist, dass er die Punktionsnadel (16) abdeckt und das distale Ende der Punktionsnadel (16) abdichtet, und ein in dem Durchgangsloch (64) untergebrachten Teil und ein distales Ende aufweist, das von dem Durchgangsloch (64) freiliegt; und
ein Vorspannelement (36), das in dem Durchgangsloch (64) untergebracht ist und das äußere Zylinderelement (32) in Bezug auf das innere Element (30) vorspannt, wobei, wenn die Punktionsnadel-Schutzanordnung (10) an der Spritze (102) angebracht ist, das äußere Zylinderelement (32) in eine Anfangsposition, um mindestens einen Teil der Punktionsnadel (16) abzudecken, in eine proximale Endposition, um den Teil der Punktionsnadel (16) durch relative Bewegung in einer proximalen Endrichtung in Bezug auf das innere Element (30) freizulegen, und in eine Verriegelungsposition, um das distale Ende der Punktionsnadel (16) durch Bewegung von der proximalen Endposition in einer distalen Endrichtung in Bezug auf das innere Element (30) abzudecken, beweglich ist,
wobei das innere Element (30) so konfiguriert ist, dass es sich relativ zu dem äußeren Zylinderelement (32) durch Bewegung des Nockenvorsprungs (42a, 42b) in dem Führungskanal (72a, 72b) dreht, während sich das äußere Zylinderelement (32) von der Anfangsposition in die Verriegelungsposition bewegt, und so konfiguriert ist, dass es eine Bewegung des äußeren Zylinderelements (32) in der proximalen Richtung in Bezug auf das innere Element (30) einschränkt, wenn das äußere Zylinderelement (32) in die Verriegelungsposition bewegt wird, und
wobei der Nadelschutzkappenkörper (34) einen Druckabschnitt (54) aufweist, der so konfiguriert ist, dass er das innere Element (30) in Richtung des Nadelhalteabschnitts (18) drückt, indem er an den Anschlagabschnitt (45) des inneren Elements (30) anstößt,
**dadurch gekennzeichnet, dass**
einen Hakenabschnitt (62), der so konfiguriert ist, dass er von dem abgestuften Abschnitt (70) des äußeren Zylinderelements (32) eingehakt wird, und
der Nadelschutzkappenkörper (34) ein punktiertes Element (46), das aus einem elastischen Körper gebildet ist, der einen punktierten Abschnitt (46b), der so konfiguriert ist, dass er mit dem distalen Ende der Punktionsnadel (16) punktiert werden kann, und einen Abdeckabschnitt (46a) umfasst, der sich von dem punktierten Abschnitt (46b) in proximaler Richtung erstreckt, um so eine Außenfläche der Punktionsnadel (16) abzudecken; und ein starres Element (48) umfasst, das konfiguriert ist, um an einer Außenseite des punktierten Elements (46) angebracht zu werden und eine höhere Steifigkeit als das punktierte Element (46) aufweist, wobei der Druckabschnitt (54) in dem starren Element (48) vorgesehen ist.

2. Punktionsnadel-Schutzanordnung (10) nach Anspruch 1, wobei mindestens einer von dem abgestuften Abschnitt (70) und dem Hakenabschnitt (62) elastisch verformbar ist.

3. Punktionsnadel-Schutzanordnung (10) nach Anspruch 1 oder 2, wobei das starre Element (48) eine Vielzahl von säulenförmigen Abschnitten (56), die sich entlang der Richtung der Mittelachse erstrecken, und einen Schlitz (60) aufweist, welcher zwischen zwei benachbarten säulenförmigen Abschnitten unter der Vielzahl von säulenförmigen Abschnitten (56) ausgebildet ist, um das punktierte Element (46) freizulegen.

4. Punktionsnadel-Schutzanordnung (10) nach Anspruch 3, wobei der Hakenabschnitt (62) an einer Außenfläche des säulenförmigen Abschnitts (56) ausgebildet ist.

5. Punktionsnadel-Schutzanordnung (10) nach einem der Ansprüche 1 bis 4, wobei das punktierte Element (46) einen Abschnitt mit engem Kontakt an einem proximalen Ende des Abdeckabschnitts (46a) aufweist, wobei der Abschnitt mit engem Kontakt so konfiguriert ist, dass er in engen Kontakt mit einem distalen Ende des Nadelhalteabschnitts (18) gebracht werden kann, und
wobei, wenn die Punktionsnadel-Schutzanordnung (10) an der Spritze (102) angebracht ist, das punktierte Element (46) zwischen dem distalen Ende des Nadelhalteabschnitts (18) und dem Hakenabschnitt (62) derart zusammengedrückt wird, dass eine luftdichte Abdichtung zum Abdichten des Abdeckabschnitts (46a) zwischen dem Abschnitt mit engem Kontakt und dem distalen Ende des Nadelhalteabschnitts (18) gebildet wird.

6. Punktionsnadel-Schutzanordnung (10) nach einem der Ansprüche 1 bis 5, wobei
ein erster Eingriffsabschnitt (44a, 44b) auf dem inneren Element (30) ausgebildet ist, und
ein zweiter Eingriffsabschnitt (54) an dem Nadelschutzkappenkörper (34) ausgebildet ist, wobei der zweite Eingriffsabschnitt mit dem ersten Eingriffsabschnitt (44a, 44b) entlang der Mittelachse in Eingriff steht.

7. Punktionsnadel-Schutzanordnung (10) nach einem der Ansprüche 1 bis 6, wobei
der Hakenabschnitt (62) einen sich verjüngenden Abschnitt aufweist, der von einer Außenfläche des Nadelschutzkappenkörpers (34) vorsteht und dessen Vorsprungshöhe allmählich zu einem distalen Ende hin zunimmt, und
ein distales Ende des sich verjüngenden Abschnitts konfiguriert ist, um an einem proximalen Ende des abgestuften Abschnitts (70) eingehakt zu werden.

8. Punktionsnadel-Schutzanordnung (10) nach einem der Ansprüche 1 bis 7, wobei
der abgestufte Abschnitt (70) an einer Innenfläche der Seitenwand (66) des äußeren Zylinderelements (32) vorgesehen ist, und
der Hakenabschnitt (62) sich als mindestens drei Hakenabschnitte an der Außenfläche des Nadelschutzkappenkörpers (34) ausbildet und einen Stützabschnitt (63) aufweist, der an einer inneren Umfangswand des abgestuften Abschnitts (70) anliegt.

9. Punktionsnadel-Schutzanordnung (10) nach einem der Ansprüche 1 bis 8, wobei
der abgestufte Abschnitt (70) einen distalen Endsitzabschnitt umfasst, der von der Seitenwand (66) in der Nähe einer distalen Endöffnung des äußeren Zylinderelements (32) vorsteht, wobei der distale Endsitzabschnitt ein distales Ende des Vorspannelements (36) an einer Außenseite als ein Teil trägt, das mit dem Hakenabschnitt (62) in Eingriff zu bringen ist.

10. Punktionsnadel-Schutzanordnung (10) nach einem der Ansprüche 1 bis 9, wobei
ein Flanschabschnitt (90) an einem Teil des Nadelschutzkappenkörpers (34) ausgebildet ist, der von dem Durchgangsloch (64) freigelegt ist, wobei der Flanschabschnitt in einer Richtung rechtwinklig zu der Mittelachse vorsteht.

11. Spritzenanordnung (100), umfassend:
die Punktionsnadel-Schutzanordnung (10) nach einem der Ansprüche 1 bis 10; und
eine Spritze (102), an welcher die Punktionsnadel-Schutzanordnung (10) angebracht ist.

12. Spritzenanordnung (100) nach Anspruch 11, wobei die Spritze (102) eine vorgefüllte Spritze (102) ist, die umfasst:
ein flüssiges Arzneimittel (104), das in den Zylinderabschnitt (12) eingefüllt ist;
eine Dichtung (106), die verschiebbar in den Zylinderabschnitt (12) eingesetzt ist; und
einen Schieber (108), um die Dichtung (106) zu betätigen.

13. Verfahren zur Herstellung einer Spritzenanordnung (100), um die Spritzenanordnung (100) nach Anspruch 11 zu erhalten, indem die Punktionsnadel-Schutzanordnung (10) nach einem der Ansprüche 1 bis 10 an einer Spritze (102) angebracht wird, wobei das Verfahren zur Herstellung der Spritzenanordnung (100) umfasst:
Zusammenbauen der Punktionsnadel-Schutzanordnung (10) im Voraus, bevor das innere Element (30) an dem Nadelhalteabschnitt (18) angebracht wird;
Bewirken, dass sich ein proximales Ende der Punktionsnadel-Schutzanordnung (10) der Spritze (102) von einer distalen Endseite der Spritze (102) her nähert;
**gekennzeichnet durch**
das Drücken des Nadelschutzkappenkörpers (34) in Richtung des proximalen Endes, um das innere Element (30) über einen Anschlag zwischen dem Druckabschnitt (54) des starren Elements (48) und dem Anschlagabschnitt (45) in Richtung des Nadelhalteabschnitts (18) zu drücken.

## Revendications

1. Ensemble de protection d'aiguille de ponction (10) configuré pour être monté sur une seringue (102) qui comprend : une portion cylindre (12) configurée pour être remplie d'un médicament liquide (104) ; une portion de maintien d'aiguille (18) qui est fournie au niveau d'une extrémité distale de la portion cylindre (12) ; et une aiguille de ponction creuse (16) qui est maintenue par la portion de maintien d'aiguille (18) et comporte une extrémité distale faisant saillie distalement par rapport à la portion de maintien d'aiguille (18) et une extrémité proximale communiquant avec l'intérieur de la portion cylindre (12), et configurée pour protéger l'aiguille de ponction (16), l'ensemble de protection d'aiguille de ponction (10) comprenant :
un élément intérieur (30) qui est configuré pour pouvoir être monté sur la portion de maintien d'aiguille (18), l'élément intérieur comportant une saillie de came (42a, 42b) et une portion butée (45) ;
un élément cylindre extérieur (32) formé d'un corps creux qui comporte une paroi latérale (66) formant un trou traversant (64) s'étendant d'une extrémité distale à une extrémité proximale, un passage de guidage (72a, 72b) formé dans la paroi latérale (66), une portion étagée (70) formée dans la paroi latérale (66), et un axe central, l'élément intérieur (30) configuré pour être reçu dans le trou traversant (64), la saillie de came (42a, 42b) de l'élément intérieur (30) configurée pour être mobile dans le passage de guidage (72a, 72b), et l'élément cylindre extérieur (32) configuré pour être mobile par rapport à l'élément intérieur (30) le long de l'axe central ;
un corps de capuchon d'aiguille (34) formé d'un corps cylindrique qui comporte une extrémité distale fermée et une extrémité proximale ouverte, le corps de capuchon d'aiguille configuré pour couvrir l'aiguille de ponction (16) et sceller l'extrémité distale de l'aiguille de ponction (16), et comportant une partie reçue dans le trou traversant (64) et une extrémité distale exposée depuis le trou traversant (64) ; et
un élément de sollicitation (36) qui est reçu dans le trou traversant (64) et sollicite l'élément cylindre extérieur (32) par rapport à l'élément intérieur (30), dans lequel, lorsque l'ensemble de protection d'aiguille de ponction (10) est fixé à la seringue (102), l'élément cylindre extérieur (32) est mobile vers une position initiale pour couvrir au moins une partie de l'aiguille de ponction (16), une position d'extrémité proximale pour exposer la partie de l'aiguille de ponction (16) par déplacement relatif dans une direction d'extrémité proximale par rapport à l'élément intérieur (30), et une position de verrouillage pour couvrir l'extrémité distale de l'aiguille de ponction (16) par déplacement depuis la position d'extrémité proximale dans une direction d'extrémité distale par rapport à l'élément intérieur (30),
dans lequel l'élément intérieur (30) est configuré pour pivoter par rapport à l'élément cylindre extérieur (32) par le mouvement de la saillie de came (42a, 42b) dans le passage de guidage (72a, 72b) alors que l'élément cylindre extérieur (32) se déplace de la position initiale à la position de verrouillage, et configuré pour restreindre un mouvement de l'élément cylindre extérieur (32) dans la direction proximale par rapport à l'élément intérieur (30) lorsque l'élément cylindre extérieur (32) se déplace dans la position de verrouillage, et
dans lequel le corps de capuchon d'aiguille (34) comporte une portion de pression (54) qui est configurée pour pousser l'élément intérieur (30) vers la portion de maintien d'aiguille (18) en butant contre la portion butée (45) de l'élément intérieur (30),
**caractérisé en ce que**
une portion crochet (62) qui est configurée pour être accrochée par la portion étagée (70) de l'élément cylindre extérieur (32), et
le corps de capuchon d'aiguille (34) comprend un élément percé (46) formé d'un corps élastique qui comporte une portion percée (46b) configurée pour être percée avec l'extrémité distale de l'aiguille de ponction (16) et une portion de couverture (46a) s'étendant à partir de la portion percée (46b) dans la direction proximale pour couvrir une surface extérieure de l'aiguille de ponction (16) ; et un élément rigide (48) qui est configuré pour être monté sur un extérieur de l'élément percé (46) et comporte une plus grande rigidité que l'élément percé (46), et la portion de pression (54) est fournie dans l'élément rigide (48).

2. Ensemble de protection d'aiguille de ponction (10) selon la revendication 1, dans lequel au moins l'une de la portion étagée (70) et de la portion crochet (62) est élastiquement déformable.

3. Ensemble de protection d'aiguille de ponction (10) selon la revendication 1 ou 2, dans lequel l'élément rigide (48) comporte une pluralité de portions colonnaires (56) s'étendant le long de la direction de l'axe central et une fente (60) formée entre deux portions colonnaires adjacentes parmi la pluralité de portions colonnaires (56) pour exposer l'élément percé (46) .

4. Ensemble de protection d'aiguille de ponction (10) selon la revendication 3, dans lequel la portion crochet (62) est formée sur une surface extérieure de la portion colonnaire (56).

5. Ensemble de protection d'aiguille de ponction (10) selon l'une quelconque des revendications 1 à 4, dans lequel l'élément percé (46) comporte une portion contact étroit au niveau d'une extrémité proximale de la portion de couverture (46a), la portion contact étroit étant configurée pour être mise en contact étroit avec une extrémité distale de la portion de maintien d'aiguille (18), et
dans lequel, lorsque l'ensemble de protection d'aiguille de ponction (10) est monté sur la seringue (102), l'élément percé (46) est compressé entre l'extrémité distale de la portion de maintien d'aiguille (18) et la portion crochet (62) de sorte qu'un joint étanche à l'air pour sceller la portion de couverture (46a) soit formé entre la portion contact étroit et l'extrémité distale de la portion de maintien d'aiguille (18).

6. Ensemble de protection d'aiguille de ponction (10) selon l'une quelconque de revendications 1 à 5, dans lequel
une première portion de mise en prise (44a, 44b) est formée sur l'élément intérieur (30), et
une seconde portion de mise en prise (54) est formée sur le corps de capuchon d'aiguille (34), la seconde portion de mise en prise mise en prise avec la première portion de mise en prise (44a, 44b) le long de l'axe central.

7. Ensemble de protection d'aiguille de ponction (10) selon l'une quelconque des revendications 1 à 6, dans lequel
la portion crochet (62) comporte une portion conique qui fait saillie à partir d'une surface extérieure du corps de capuchon d'aiguille (34) et dont une hauteur de saillie augmente progressivement vers une extrémité distale, et
une extrémité distale de la portion conique est configurée pour être accrochée par une extrémité proximale de la portion étagée (70).

8. Ensemble de protection d'aiguille de ponction (10) selon l'une quelconque des revendications 1 à 7, dans lequel
la portion étagée (70) est fournie sur une surface intérieure de la paroi latérale (66) de l'élément cylindre extérieur (32), et
la portion crochet (62) prend la forme d'au moins trois portions crochet sur la surface extérieure du corps de capuchon d'aiguille (34) et comporte une portion support (63) qui bute contre une paroi périphérique de la portion étagée (70).

9. Ensemble de protection d'aiguille de ponction (10) selon l'une quelconque des revendications 1 à 8, dans lequel
la portion étagée (70) comporte une portion siège d'extrémité distale qui fait saillie à partir de la paroi latérale (66) dans le voisinage d'une ouverture d'extrémité distale de l'élément cylindre extérieur (32), la portion siège d'extrémité distale supportant une extrémité distale de l'élément de sollicitation (36) au niveau d'un côté extérieur par rapport à une partie à mettre en prise avec la portion crochet (62).

10. Ensemble de protection d'aiguille de ponction (10) selon l'une quelconque des revendications 1 à 9, dans lequel
une portion rebord (90) est formée au niveau d'une partie du corps de capuchon d'aiguille (34) exposée depuis le trou traversant (64), la portion rebord faisant saillie dans une direction orthogonale à l'axe central.

11. Ensemble seringue (100) comprenant :
l'ensemble de protection d'aiguille de ponction (10) selon l'une quelconque des revendications 1 à 10 ; et
une seringue (102) sur laquelle l'ensemble de protection d'aiguille de ponction (10) est monté.

12. Ensemble seringue (100) selon la revendication 11, dans lequel
la seringue (102) est une seringue préremplie (102) qui comporte :
un médicament liquide (104) dont est remplie la portion cylindre (12) ;
un joint d'étanchéité (106) inséré de manière coulissante dans la portion cylindre (12) ; et
un poussoir (108) pour actionner le joint d'étanchéité (106).

13. Procédé de fabrication d'un ensemble seringue (100) pour obtenir l'ensemble seringue (100) selon la revendication 11 en montant l'ensemble de protection d'aiguille de ponction (10) selon l'une quelconque des revendications 1 à 10 sur une seringue (102), le procédé de fabrication de l'ensemble seringue (100) comprenant :
l'assemblage de l'ensemble de protection d'aiguille de ponction (10) à l'avance avant le montage de l'élément intérieur (30) sur la portion de maintien d'aiguille (18) ;
la provocation du rapprochement d'une extrémité proximale de l'ensemble aiguille de ponction (10) de la seringue (102) à partir d'un côté extrémité distale de la seringue (102) ;
**caractérisé par**
la pression du corps de capuchon d'aiguille (34) dans la direction de l'extrémité proximale pour pousser l'élément intérieur (30) vers la portion de maintien d'aiguille (18) via une butée entre la portion de pression (54) de l'élément rigide (48) et la portion butée (45).
